Europäisches Patentamt

(19) European Patent Office

Office européen des brevets

(11) Veröffentlichungsnummer: **0 141 358**
**B1**

(12) # EUROPÄISCHE PATENTSCHRIFT

(45) Veröffentlichungstag der Patentschrift:
25.11.87

(21) Anmeldenummer: 84112685.7

(22) Anmeldetag: 20.10.84

(51) Int. Cl.⁴: **C 07 C 29/80,** C 07 C 31/22, C 07 C 29/76

(54) Verfahren zur verbesserten destillativen Aufarbeitung von Glycerin.

(30) Priorität: 28.10.83 DE 3339051

(43) Veröffentlichungstag der Anmeldung:
15.05.85 Patentblatt 85/20

(45) Bekanntmachung des Hinweises auf die Patenterteilung:
25.11.87 Patentblatt 87/48

(84) Benannte Vertragsstaaten:
BE DE FR IT NL

(56) Entgegenhaltungen:
FR-A-1 138 886
FR-A-1 557 590

Ullmann, Band 12 (1976), 369
Kirk-Othmer, vol. 11 (1980), 925

(73) Patentinhaber: Henkel Kommanditgesellschaft auf Aktien, Postfach 1100 Henkelstrasse 67, D-4000 Düsseldorf- Holthausen (DE)

(72) Erfinder: Brockmann, Rolf, Dr., Falkenweg 10, D-4018 Langenfeld (DE)
Erfinder: Jeromin, Lutz, Dr., Am Bandsbusch 88, D-4010 Hilden (DE)
Erfinder: Johannisbauer, Wilhelm, Dr., Erich-Kästner- Strasse 26, D-4006 Erkrath 1 (DE)
Erfinder: Meyer, Helmut, Dr., Bennrather- Schloss-Allee 71, D-4000 Düsseldorf- Benrath (DE)
Erfinder: Michel, Otto, Am Bendenbusch 11, D-4018 Langenfeld (DE)
Erfinder: Plachenka, Jürgen, Dr., Kloppstock 1, D-4020 Mettmann (DE)

**Beschreibung**

Die Erfindung betrifft ein Verfahren zur verbesserten destillativen Aufarbeitung von Glycerin, das durch Umesterung, Spaltung oder Verseifung nativer Fette und Öle erhalten wird, durch Alkalisierung der glycerinhaltigen Rohmischung unter Luftoxidation, Verdampfung der Mischung in einem Dünnschichtverdampfer mit Nachdestillation des Rückstandes, Rektifikation und erneute Verdampfung in einer durch druckverlustarme Einbauten gekennzeichneten Packungskolonne mit einem Fallfilmverdampfer mit innerer und äußerer Partialkondensation unter Abtrennung nicht erwünschter Mischungsbestandteile, Bleichung des erhaltenen Glycerins mit Aktivkohle und Abtrennen des Bleichmittels auf an sich bekannten Wegen. Ziel der Aufarbeitung ist die Gewinnung eines Reinglycerins für die Verwendung im Lebensmittelbereich, in der Kosmetik- und Pharmaindustrie, das z. B. der Europharm III Vorschrift entspricht, oder eines Glycerins für technische Lieferbedingungen nach DIN 55 967, o.a.

Glycerin entsteht bei der Umesterung, Spaltung oder Verseifung nativer Öle und Fette z. B. durch Verseifung mit Alkali oder durch Hochdruckhydrolyse, und enthält, je nach Verseifungsverfahren, größere Mengen an Wasser, anorganische Salze, Fette, niedermolekulare organische Verbindungen sowie auch höhere Glycerinoligo- bzw. -polymere.

Die im Rohglycerin anfallenden organischen Verunreinigungen bestehen in erster Linie aus Fettstoffen und durch bakterielle oder chemische Zersetzung entstandenen glycerinähnliche Verbindungen, wie z. B. 1,2- und 1,3-Propandiol, oder auch anderen Komponenten, wie Glycerinmethylether, die sich in ihren physikalischen Eigenschaften (Siedepunkt, Brechungsindex) kaum von Glycerin unterscheiden und den Reinigungsvorgang erheblich stören, da sie in herkömmlichen Kolonnen mit niedriger theoretischer Bodenzahl zusammen mit Glycerin destilliert werden. Diese Begleitstoffe sind zum Teil für eine unerwünschte Färbung und geringe Farbstabilität des Rohglycerins verantwortlich; ihre Abtrennung ist also unbedingt nötig.

Für die aus der alkalischen Verseifung stammende Unterlauge und das bei der Hochdruckhydrolyse von Fetten und Ölen anfallende Süßwasser gibt Ullmanns Encyclopädie der technischen Chemie, 4. Auflage, Band 12, Weinheim, New York 1976, Seite 369 folgende Zusammensetzung an:

| | Unterlauge Massen-% | Süßwasser Massen-% |
|---|---|---|
| Glycerin | 82-84 | 88-93 |
| anorganische Salze | 8-9,5 | 0 |
| organische Begleitstoffe | 0,5-2 | 0,5-1,0 |
| 1,3-Propandiol | 0,1-0,5 | 0,1-0,5 |
| Stickstoff | 0,01-0,04 | 0,02-0,04 |
| Arsen | 0,1-0,5 ppm | 0,1 ppm |

Nach Ullmanns Encyclopädie, loc. cit. können alle ionischen Verunreinigungen aus der verdünnten Glycerin-Lösung durch kontinuierlichen Kationenaustausch entfernt werden. Anschließend kann die Glycerin-Lösung ohne fraktionierte Destillation zu einem Produkt hoher Qualität entwässert werden. Zur Herstellung von hochreinem Glycerin, das den Anforderungen der Nahrungsmittelindustrie entspricht, wird das rohe Glycerin destilliert und mit Aktivkohle behandelt. Da Glycerin bei etwa 180°C sich zu zersetzen beginnt, arbeitet man bei 6,5 mbar und 150-170°C mit einer Kombination aus Dampfdestillation und fraktionierter Destillation.

Nach Kirk-Othmer, Encyclopaedia of Chemical Technology, Third Edition, Volume 11, New York-Chichester-Brisbane-Toronto 1980, Seite 925, wird bei der Destillation von Glycerin eine Kombination aus Vakuum- und Wasserdampfdestillation benutzt, in der die Dämpfe aus der Destillationsanlage durch eine Reihe von Kondensoren geleitet werden, wobei relativ reines Glycerin bei hohen Temperaturen kondensiert wird. In einigen modernen Anlagen wird das rohe Glycerin in den unteren Abschnitten der Destillationsapparatur bei viel niedrigeren Temperaturen verdampft, als dies in den älteren Anlagen möglich ist. Auf diese Weise werden durch Polymerisation und Zersetzung verursachte Glycerinverluste vermieden. Glycerin, das für die Lebensmittelindustrie bestimmt ist, wird nach Kirk-Othmer, loc. cit., zwecks Bleichung und Desodorierung bei 74-79°C mit 1-2 % Aktivkohle und Filtrierhilfsmittel versetzt, 1-2 Stunden lang gerührt und schließlich bei dieser Temperatur filtriert.

Alle technisch durchgeführten Aufarbeitungsverfahren für Glycerin aus natürlichen Fetten und Ölen haben gemeinsam, daß zur Herstellung von hochreinem Glycerin eine Rohmischung erforderlich ist, deren Gehalt an organischen Begleitstoffen (Mongggehalt) ⩽ 1 Gew.-% ist und aus der Fett-, Seifen- und andere organische Bestandteile vorher entfernt worden sind. Hierzu werden die Fettstoffe in einer aufwendigen chemischen Vorreinigungsstufe mit Kalkmilch oder nach dem Kalk-Soda-Verfahren verseift und soweit wie möglich in Form von Seifen abfiltriert. Die weitere Abtrennung, insbesondere von anorganischen Verunreinigungen, erfolgt auf destillativem Wege, wobei in der Rektifikations-Stufe Kolonnen verwendet werden, deren Einbauten einen hohen Druckverlust bedingen. Um eine ausreichende Trennung der verschiedenen Komponenten zu erzielen, müssen die Sumpftemperaturen entsprechend hoch sein; diesem Verfahrensparameter sind dadurch Grenzen gesetzt, daß Glycerin bei Temperaturen um 180°C beginnt, Wasser abzuspalten und sich zu zersetzen und zu polymerisieren.

Je nach Salzgehalt erfolgt die Aufarbeitung von Rohglycerin auf verschiedenen Wegen: Salzarmes Rohglycerin kann unmittelbar über Ionenaustauscher gereinigt werden, während salzreiches Rohglycerin destillativ vorbehandelt werden muß, um das Salz aus dem Rohglycerin zu entfernen. Damit Reinglycerin der erforderlichen Handelsqualität erhalten wird, schließt sich an die Destillation eine Behandlung mit Ionenaustauschern an, wozu das konzentrierte Glycerin wieder verdünnt werden muß. Beide Verfahrenswege sind apparativ und energetisch sehr aufwendig, lassen sich nicht immer kontinuierlich durchführen und haben erhebliche Verluste an Glycerin zur Folge. Um Glycerin auch von höhersiedenden Begleitstoffen destillativ zu trennen, muß die Mischung zudem einer hohen thermischen Belastung unterworfen werden, was weitere Glycerinverluste und das Auftreten weiterer Mengen an Zersetzungeprodukten zur Folge hat.

Die vorliegende Erfindung stellt sich die Aufgabe, ein verbessertes Verfahren zu schonenden Destillation von Glycerin zur Verfügung zu stellen, in dem in kontinuierlicher Arbeitsweise Glycerin auf der Basis nativer Fette und Öle von seinen Begleitstoffen, insbesondere Salzen, Wasser und die chemischen und physikalischen Eigenschaften beeinflussenden organischen Begleitsubstanzen, befreit wird, ohne daß Glycerin einer hohen thermischen Belastung unterworfen werden muß, wobei die Ausbeuten bei 90 % und höher liegen und die Qualität mindestens den o.g. Standard erreichen sollte.

Die vorliegende Erfindung betrifft somit ein Verfahren zur verbesserten destillativen Aufarbeitung von Glycerin, das durch Umesterung, Spaltung oder Verseifung nativer Fette und Öle erhalten wird, durch aufeinanderfolgende Verfahrensschritte der Verdampfung, Rektifikation, Bleichung und Abtrennung des Bleichmittels, das dadurch gekennzeichnet ist, daß

a) Rohglycerin mit einem Wassergehalt bis ca. 10 Gew.-% ohne Vortrocknung und chemische Vorreinigung in einem Rührkessel bei 90 bis 100°C eine Stunde mit wässriger Alkalihydroxidlösung alkalisiert und mit 10 Nm$^3$ Luft/m$^3$ Rohglycerin versetzt wird;

b) die so erhaltene Mischung in einem Dünnschichtverdampfer mit mechanisch angetriebenen Wischerblättern bei 10 bis 20 mbar und 165 bis 180°C destilliert wird, wobei die schwersiedenden Bestandteile über eine Schleuse in eine Rückstandsvorläge ausgetragen werden, wo eine Nachdestillation erfolgt;

c) die Brüden aus dem Verdampfer über einen Tropfenabscheider in das Unterteil einer Kolonne mit druckverlustarmen Einbauten gelangen und diese durchströmen, wobei die Einbauten zu einem Druckverlust von höchstens 1 mbar/theoretischer Trennstufe bei einer vergleichbaren Luftgeschwindigkeit von mindestens 2 m/s führen und mindestens 7 theoretische Trennstufen vorliegen;

d) die Brüden der Kolonne bei einem Kopfdruck 5 bis 10 mbar in einem Kondensator bei 88 bis 90°C partiell niedergeschlagen, der Rest in einem zweiten Kondensator bei 20 bis 30°C kondensiert werden, wobei das Kondensat des zweiten Kondensators ca. 1 % der eingesetzten Rohglycerinmenge entspricht;

e) das Kondensat aus dem ersten Kondensator um 30 bis 50°C gekühlt und als Rücklauf auf den Kolonnenkopf gegeben wird;

f) auf einer Höhe von ungefähr 1/3 der Kolonnenhöhe der Produkthauptlauf als Seitenstrom kontinuierlich flüssig aus der Kolonne entnommen wird, wobei ein Teilstrom unmittelbar unter der Entnahmestelle mit derselben Temperatur oder um 20 bis 40°C gekühlt in die Kolonne zurückgeführt wird;

g) der Rest des Produkthauptlaufs auf 80 bis 90°C gekühlt und nach Zugabe von Aktivkohle in einer Menge von 0,1 bis 0,3 Gew.-% in einem Behälter bei 80 bis 90°C für 15 bis 30 Minuten unter Stickstoff gerührt und zur Abtrennung des Bleichmittels durch eine Rahmenfilterpresse geführt wird;

h) der Rücklauf aus der Entnahme des Produkthauptlaufs in einem Fallfilmverdampfer bei Temperaturen von 150 bis 180°C verdampft und die Brüden in den Sumpf der Kolonne zurückgeführt werden und

i) ein kleiner Teilstrom von 1 % der eingesetzten Rohglycerinmenge kontinuierlich aus dem Sumpf der Kolonne entnommen wird.

j) die im zweiten Kondensator niedergeschlagenen Brüden gegebenenfalls gemeinsam mit dem Sumpfablauf der Kolonne in einem nochmaligen Durchlauf aufgearbeitet bzw. teilweise rückgeführt werden.

Das Verfahren der vorliegenden Erfindung erlaubt die kontinuierliche Reinigung sowohl salzreicher als auch salzarmer bzw. salzfreier Rohglycerine. Es ist auch auf Rohglycerine aus sogenannten Unterlaugen aus der alkalischen Verseifung, die einen hohen Anteil an Fettverbindungen (Monggehalt $\geqslant$ 3 Gew.-%), einen hohen Salzanteil ($\geqslant$ 6 Gew.-%) und einen Wasseranteil bis 10 Gew.-% aufweisen, anwendbar. Eine chemische Vorreinigung ist auch bei schlechten Rohqualitäten und höchsten Qualitätsanforderungen nach Europharm III nicht erforderlich; des weiteren muß lediglich bis ca. 10 Gew.-% Wasser vorgetrocknet werden, was noch bei Normaldurck ohne Anlegen von Vakuum geschehen kann. Durch die Anwendung neuartiger Technologien im Destillations- und Rektifikationsbereich, wie z. B. die Kombination von Dünnschicht- und Fallfilmverdampfern und Packungskolonnen mit druckverlustarmen Einbauten, werden trotz relativ niedriger Sumpftemperaturen hohe Trennnleistungen möglich, was eine sehr schonende Behandlung des Glycerins ermöglicht und Verluste durch Zersetzungsreaktionen verhindert. Durch die Integration verschiedener Verfahrensschritte, wie Hauptlauf-, Vorlauf-, Rückstands- und Sumpfabtrennung in einer Verfahrensstufe erfolgt die Glycerinabtrennung sowohl unter apparativen als auch energetischen Gesichtspunkten optimal. Die Ausbeuten belaufen sich auf 90 bis 95 % der eingesetzten Glycerinmenge; die Qualität des Produktes ist entsprechend den Anforderungen einstellbar, wobei - in Abhängigkeit von der Qualität des Rohmaterials - die Ausbeute selbst für hochreines Glycerin noch über 90 % liegt.

Die Packungskolonne, die in dem erfindungsgemäßen Verfahren eingesetzt wird, ist mit 2 Verdampfern ausgestattet und liefert in einem Verfahrensschritt ein bidestilliertes Endprodukt. Die druckverlustarmen Einbauten ermöglichen eine hohe Trennstufenzahl in diesem Verfahrensschritt und führen zur schonenden Abtrennung der Fette und der glycerinähnlichen Substanzen (1,2- und 1,3-Propandiol sowie Glycerinmethylether), was in Kolonnen herkömmlicher Bauart aufgrund einer zu niedrigen Trennstufenzahl

3

infolge des hohen Druckverlusts je Trennstufe und der damit verbundenen hohen thermischen Belastung des Glycerins nicht möglich war.

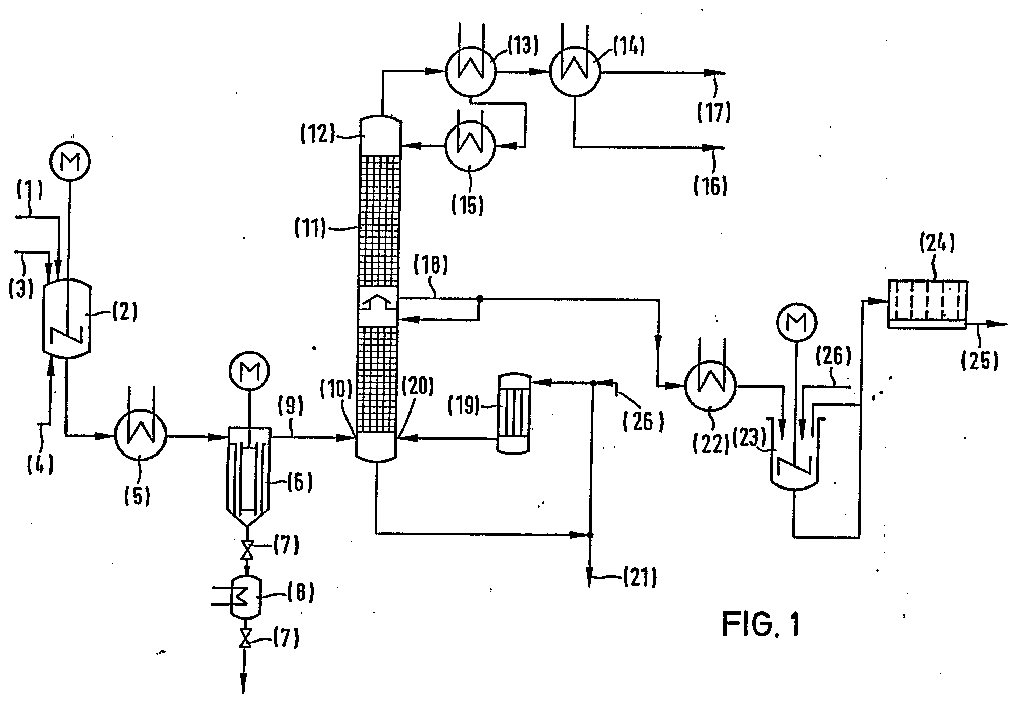

Figur 1 zeigt ein Fließschema des erfindungsgemäßen Verfahrens zur schonenden destillativen Aufarbeitung von Glycerin. Die in der Figur enthaltenen Bezugszeichen werden in der nachfolgenden detaillierten Verfahrensbeschreibung erläutert.

Die Rohglycerin-Mischung wird bei (1) in einen Rührkessel (2) gefüllt, zur Verseifung der Fette und Fettsäuren über Ansatz (3) mit 50 %iger Natronlauge oder Kalilauge versetzt und bei 90°C mindestens eine Stunde alkalisiert. Dabei wird unter Rühren bei (4) Luft zugeführt, um reduzierende Komponenten des Gemisches zu oxidieren.

Über einen Vorwärmer (5) gelangt die so behandelte Reaktionsmischung mit einer Temperatur von ca. 140°C in einen Dünnschichtverdampfer (6), in dem das Glycerin im Vakuum (max. 15 mbar) unter mechanischer Rührung schonend eingedampft wird.

Der die bei der Alkalisierung gebildeten Seifen, Salze und polymere Glycerine enthaltende Rückstand wird am unteren Ende des Dünnschichtverdampfers über einen beheizten Kugelhahn (7) in eine ebenfalls beheizte Vorlage (8) ausgetragen, in der durch höhere Temperaturen ein zusätzliches "Ausquetschen" des Rückstandes erfolgt. Das Entleeren der Vorlage (8) im kontinuierlichen Betrieb erfolgt nach Absperren des oberen Kugelhahns (7) und Belüften durch den unteren, ebenfalls beheizten Kugelhahn. Anschließend wird die Vorlage über eine Ringpumpe vorevakuiert und die Verbindung zwischen Verdampfer (6) und beheizter Rückstandsvorlage (8) wiederhergestellt.

Die Brüden aus dem Dünnschichtverdampfer (6) gelangen durch ein beheiztes Brüdenrohr (9) bei (10) in die Sumpfblase der Packungskolonne (11). In dem beheizten Brüdenrohr (9) sind Abscheidehilfen angebracht, die ein Mitreißen von Flüssigkeitströpfchen aus dem Verdampfer (6) unterbinden.

Die Brüden werden bei ihrem Aufstieg durch die Packungskolonne (11) rektifiziert. Die Kolonne ist mit neuartigen, druckverlustarmen Einbauten versehen, so daß eine hohe Trennstufenzahl erreicht wird, ohne daß Glycerin durch eine hohe Temperaturbelastung in der Sumpfblase zersetzt wird. Die druckverlustarmen Einbauten führen bei einer vergleichbaren Luftgeschwindigkeit von mindestens 2 m/s zu einem Druckverlust von maximal 1 mbar/theoretische Trennstufe, wobei mindestens 7 theoretische Trennstufen vorliegen.

Die Brüden der oberen Trennzone werden im Kondensator I (13) mit ca. 85°C bei max. 10 mbar Kopfdruck partiell niedergeschlagen (Dephlegmator). Die entstandene flüssige Phase wird im Rücklaufkühler (15) abgekühlt und auf den Kopf (12) der Kolonne (11) zurückgegeben (innere und äußere Partialkondensation). Die leichter siedenden Komponenten der vom Kolonnenkopf (12) abgehenden Brüden kondensieren in einem zweiten Kondensator (14) und werden als Vorlauf bei (16) abgezogen. Im Vorlauf sind neben Glycerin hauptsächlich Ester, Fette und glycerinähnliche Substanzen zu finden. Die nicht kondensierbaren Brüden, hauptsächlich Wasser, gehen unkondensiert in die Vakuumanlage (17).

Schwer siedende Stoffe, wie dimeres Glycerin und farbtragende Substanzen, lassen sich durch mindestens zwei theoretische Trennstufen im unteren Drittel der Kolonne von den aufsteigenden Brüden abtrennen. Am oberen Ende dieses Bereiches der Kolonne wird bei (18) der gesamte Kolonnenrücklauf seitlich als Produkthauptlauf abgezogen. Ein Teil des Produkthauptlaufs gelangt an derselben Stelle (18) mit derselben Temperatur oder auch geringfügig unterkühlt zurück in die Kolonne. Diese Menge ist einstellbar und dient dazu, die schwersiedenden und farbtragenden Bestandteile aus dem Dampf mit gutem Produkt abzutreiben.

Der Sumpfblase am unteren Ende der Kolonne (11) wird kontinuierlich eine geringe Menge des flüssigen Gemisches entzogen, um einer zu starken Aufkonzentrierung an farbtragenden Substanzen entgegenzuwirken. Der Sumpf der Kolonne wird im Sumpffallfilmverdampfer (19) bei einer Temperatur von maximal 165°C schonend verdampft (zweite Verdampfung), wobei die Brüden bei (20) in den Kolonnensumpf zurückgegeben werden, der Rückstand als Sumpfabzug (21) gesammelt und gegebenenfalls zusammen mit dem Vorlauf (16) in einem nochmaligen Durchlauf aufgearbeitet werden kann.

Der größere Anteil des bei (18) abgezogenen Produkthauptlaufs wird in einem Kühler (22) abgekühlt und anschließend in einem Rührkessel (23) mittels Aktivkohle (26) kontinuierlich gebleicht. Die Bleichung erfolgt je nach Rohglycerinqualität mit einer Aktivkohle-Menge von 0,1 bis 0,3 % bei 80°C und einer Verweilzeit von 0,5 h, wobei die Glycerin-Kohle-Mischung von einer Reinstickstoffschicht überlagert wird. Die Kohle wird mittels Rahmenfilterpressen (24) abgetrennt.

Die dem Produkthauptlauf im Kühler (22) zur Abkühlung auf 80°C entzogene Wärme wird zur Aufheizung des Rohproduktes nach der Alkalisierung im Vorwärmer (5) vor dem Dünnschichtverdampfer (6) verwendet.

Da der bei (16) abgezogene Strom an Vorlaufkondensat noch erhebliche Mengen an Glycerin enthält, bietet sich ein nochmaliger Durchlauf des gesamten Verfahrensganges, allerdings ohne die Alkalisierung, gegebenenfalls zusammen mit dem Sumpfabzug (21) an. Die daraus im Produkthauptlauf der Kolonne resultierende Farbverschlechterung kann durch eine geringfügig höhere Aktivkohlemenge in der Bleichungsstufe ausgeglichen werden. Eine Teilrückführung des Vorlaufkondensats in den Alkalisierungsbehälter ist alternativ möglich.

Sollte der Wassergehalt im Einsatz unter 5 Gew.-% liegen, kann gegebenenfalls durch Zugabe von überhitztem Wasserdampf bei (26) auf den Kopf des Fallfilmverdampfers (19) eine Verbesserung der Farbqualität des bei (18) abgezogenen Produkts des Hauptlaufs erreicht werden.

Die als Hauptlauf abgezogenen und gebleichten Verfahrensprodukte, die bei (25) erhalten werden, weisen eine ausgezeichnete Qualität auf. Der Glyceringehalt liegt zwischen 99,8 und 99,9 %. Die Produkte sind nach der Bleichung farblos klar und weisen Hazen-Zahlen zwischen 5 und 10 auf. Im Produkt werden keine Salzrückstände mehr gefunden. Der Wassergehalt liegt unter 0,1 %. Bei derartigen Produktqualitäten wird eine Glycerinausbeute von 90 bis 95 % erhalten.

Die Erfindung wird durch die nachstehenden Beispiele näher erläutert.

Das Verfahren der vorliegenden Erfindung wurde mit Glycerinen verschiedener Herkunft gefahren:

**Beispiel 1:**

Stark salzhaltige Glycerin-Rohmischung aus der Umesterung

| | |
|---|---|
| von Kokosöl; Wassergehalt | : 2 bis 10 %, |
| Salzgehalt | : 4 bis 6 %, |
| Monggehalt | : 2 bis 3 %. |

**Beispiel 2:**

Stark salzhaltige Glycerin-Rohmischung mit hohem Anteil an Farb- und Geruchsträgern aus der Spaltung eines Rückstandes, der bei der Spaltung von Kokosöl anfällt;

| | |
|---|---|
| Wassergehalt | : 2 bis 10 %, |
| Salzgehalt | : 6 %, |
| Monggehalt | : 1,5 %. |

Mit bei den Rohglycerin-Mischungen der Beispiel 1 und 2 wurden nach Bleichung mit 0,2 bzw. 0,3 % Aktivkohle (Norit[R] und Brillonit[R] SX plus im gleichen Gewichtsverhältnis) folgende Qualitäten erzielt:

| | Qualität (1) | Qualität (2) |
|---|---|---|
| $n_D$ (20°C): | 1.4737 | 1.4737 |
| Glyceringehalt (Gew-.%): | 99,8 - 99,9 | 99,8 - 99,9 |
| Hazenzahl: | 5 | 5 - 10 |
| VW (%$Na_2O$): | 0,006 | 0,006 |
| VZ: | 0,11 | 0,11 |
| EZ (ml n-10 HCl): | 9,2 | 9,2 |
| SZ: | 0,1 | 0,1 |
| red.Stoffe | | |
| - 1. Stufe -: | farbl. klar | farbl. klar |
| - 2. Stufe -: | gelbl. grau | gelb-grau |
| Mischbarkeit Wasser: | farbl. klar | farbl. klar |
| Wassergehalt (Gew.-%): | 0,07 | 0,07 |
| Cl-: | - | - |
| $SO_4^{--}$: | - | - |
| Ausbeute (%) | 95 | 90 |

**Patentansprüche**

1. Verfahren zur verbesserten destillativen Aufarbeitung von Glycerin, das durch Umesterung, Spaltung und Verseifung nativer Fette und Öle erhalten wird, durch aufeinanderfolgende Verfahrensschritte der Verdampfung, Rektifikation, Bleichung und Abtrennung des Bleichmittels, dadurch gekennzeichnet, daß

a) Rohglycerin mit einem Wassergehalt bis ca. 10 Gew.-% ohne Vortrocknung und chemische Vorreinigung in einem Rührkessel bei 90 bis 100°C eine Stunde mit wässriger Alkalihydroxidlösung alkalisiert und mit 10 Nm$^3$ Luft/m$^3$ Rohglycerin versetzt wird;

b) die so erhaltene Mischung in einem Dünnschichtverdampfer mit mechanisch angetriebenen Wischerblättern bei 10 bis 20 mbar und 165 bis 180°C destilliert wird, wobei die schwersiedenden Bestandteile über eine Schleuse in eine Rückstandsvorlage ausgetragen werden, wo eine Nachdestillation erfolgt;

c) die Brüden aus dem Verdampfer über einen Tropfenabscheider in das Unterteil einer Kolonne mit druckverlustarmen Einbauten gelangen und diese durchströmen, wobei die Einbauten zu einem Druckverlust von höchstens 1 mbar/theoretischer Trennstufe bei einer vergleichbaren Luftgeschwindigkeit von mindestens 2 m/s führen und mindestens 7 theoretische Trennstufen vorliegen;

d) die Brüden der Kolonne bei einem Kopfdruck 5 bis 10 mbar in einem Kondensator bei 80 bis 90°C partiell niedergeschlagen, der Rest in einem zweiten Kondensator bei 20 bis 30°C kondensiert werden, wobei das Kondensat des zweiten Kondensators ca. 1 % der eingesetzten Rohglycerinmenge entspricht;

e) das Kondensat aus dem ersten Kondensator um 30 bis 50°C gekühlt und als Rücklauf auf den Kolonnenkopf gegeben wird;

f) auf einer Höhe von ungefähr 1/3 der Kolonnenhöhe der Produkthauptlauf als Seitenstrom kontinuierlich flüssig aus der Kolonne entnommen wird, wobei ein Teilstrom unmittelbar unter der Entnahmestelle mit derselben Temperatur oder um 20 bis 40°C gekühlt in die Kolonne zurückgeführt wird;

g) der Rest des Produkthauptlaufs auf 80 bis 90°C gekühlt und nach Zugabe von Aktivkohle in einer Menge von 0,1 bis 0,3 Gew.-% in einem Behälter bei 80 bis 90°C für 15 bis 30 Minuten unter Stickstoff gerührt und zur Abtrennung des Bleichmittels durch eine Rahmenfilterpresse geführt wird;

h) der Rücklauf aus der Entnahme des Produkthauptlaufs in einem Fallfilmverdampfer bei Temperaturen von 150 bis 180°C verdampft und die Brüden in den Sumpf der Kolonne zurückgeführt werden und

i) ein kleiner Teilstrom von 1 % der eingesetzten Rohglycerinmenge kontinuierlich aus dem Sumpf der Kolonne entnommen wird.

j) die im zweiten Kondensator niedergeschlagenen Brüden gegebenenfalls gemeinsam mit dem Sumpfablauf der Kolonne in einem nochmaligen Durchlauf aufgearbeitet bzw. teilweise rückgeführt werden.

2. Verfahren nach Anspruch 1, dadurch gekennzeichnet, daß gegebenenfalls 2 bis 4 kg überhitzer Wasserdampf pro 100 kg Rohglycerin über den Fallfilmverdampfer in die Kolonne geführt werden.

3. Verfahren nach Ansprüchen 1 und 3, dadurch gekennzeichnet, daß man sowohl salzhaltige als auch salzfreie Rohglycerine einsetzt.

## Claims

1. A process for the improved working up by distillation of glycerin obtained by the transesterification, splitting and saponification of native fats and oils by successive process steps of evaporation, rectification, bleaching and separation of the bleach, characterized in that

a) crude glycerin containing up to about 10 % by-weight water - without predrying and chemical prepurification - is alkalized for 1 hour at 90 to 100°C with an aqueous alkali hydroxide solution in a stirrer-equipped vessel in the presence of 10 $Nm^3$ air/$m^3$ crude glycerin;

b) the resulting mixture is distilled at 165 to 180°C/ 10 - 20 mbar in a thin-layer evaporator comprising mechanically driven wiper blades, the high-boiling constituents being discharged through a lock into a residue receiver in which they are redistilled;

c) the vapors from the evaporator pass through a drop separator into the lower part of a column comprising low-pressure-loss plates and flow through that column, the plates leading to a pressure loss of at most 1 mbar/ theoretical plate at an equivalent air velocity of at least 2 m/s and at least 7 theoretical plates being present;

d) the vapors of the column are partially diposited in a condenser at 80 to 90°C under a head pressure of from 5 to 10 mbar, the rest being condensed at 20 to 30°C in a second condenser, the condensate of the second condenser corresponding to approximately 1 % of the quantity of crude glycerin used;

e) the condensate from the first condenser is cooled by 30 to 50°C and returned to the head of the column;

f) the main product runnings is continuously removed from the column as a liquid sidestream at a height of approximately one third of the height of the column, a partial stream being returned to the column immediately beneath the point of removal either at the same temperature or cooled by 20 to 40 °C,

g) the rest of the main product runnings is cooled to 80 to 90°C and, after the addition of active carbon in a quantity of from 0.1 to 0.3 % by weight, is stirred under nitrogen in a vessel for 15 to 30 minutes at 80 to 90°C and passed through a frame filter press to separate the bleach;

h) the return stream from the removal of the main product runnings is evaporated in a falling-film evaporator at temperatures in the range from 150 to 180°C and the vapors are returned to the sump of the column;

i) a small partial streem of 1 % of the quantity of crude glycerin used is continuously removed from the sump of the column and

j) the vapors deposited in the second condenser, optionally together with the sump discharge of the column, are worked up or partly recycled in another run.

2. A process as claimed in Claim 1, characterized in that 2 to 4 kg superheated steam per 100 kg crude glycerin are optionally introduced into the column trough the falling film evaporator.

3. A process as claimed in Claims 1 and 3, characterized in that both salt-containing and salt-free crude glycerins are used.

## Revendications

1. Procédé pour améliorer le traitement distillatoire de la glycérine qui a été obtenue par transestérification, clivage et saponification de graisses et d'huiles naturelles, par des stades opératoires successifs de vaporisation, rectification, blanchiment, et séparation de l'agent de blanchiment, caractérisé en ce que

a) on alcalinise la glycérine brute ayant une teneur en eau allant jusqu'environ 10 % en poids, sans préséchage et purification chimique préalable, dans une cuve à agitation à 90 - 100°C durant une heure avec une solution aqueuse d'hydroxyde alcalin, tout en ajoutant 10 Nm$^3$ d'air/m$^3$ de glycérine brute;

b) on distille le mélange ainsi obtenu dans un évaporateur à couche mince avec lames frotteuses actionnées mécaniquement sous 10 à 20 mbars et à 165-180°C, les constituants bouillant difficilement étant déchargés au moyen d'une écluse lieu une post-distillation;

c) la vapeur chaude issue de l'évaporateur parvient en passant par un séparateur de gouttes dans la partie inférieure d'une colonne à élément incorporés pauvres en perte de pression et traverse celle-ci, en l'occurence les élements incorporés conduisant à une perte de pression au plus de 1 mbar /étage de séparation théorique à une vitesse d'air comparable d'au moins 2 m/s, avec existence d'au moins 7 étages de séparation théoriques;

d) la vapeur chaude de la colonne, à une pression de tête de 5 à 10 mbars, est précipitée partiellement dans un condenseur à 80 - 90°C, le restant étant condensé dans un deuxième condenseur à 20 - 30°C, le condensat du deuxième condenseur correspondant à environ 1 % de la quantité de glycérine brute employée;

e) le condensat du premier condenseur est refroidi de 30 à 50°C et est ajouté comme reflux en tête de la colonne;

i) à une hauteur d'environ 1/3 de la hauteur de la colonne, le courant principal de produit est prélevé sous forme de courant latéral de manière continue à l'état liquide à partir de la colonne, en l'occurence un courant partiel, directement au-dessous du point de prélévement à la même température ou refroidi de 20 à 30°C, étant renvoyé à la colonne;

g) le restant du courant principal de produit est refroidi à 80 - 90°C et après addition de charbon actif en une quantité de 0,1 à 0,38 en poids est agité dans un réservoir à 80 à 90°C pendant 15 à 30 minutes sous azote et est envoyé pour la séparation de l'agent de blanchiment à travers un filtre-presse à cadres;

h) le reflux issu du prélévement du courant principal de produit est vaporisé dans un évaporateur à film tombant à des températures de 150 à 180°C et la vapeur chaude est recyclée au bas de la colonne et

i) un petit courant partiel de 1% de la quantité de glycérine brute employée est prélevé continuellement à partir du fond de la colonne;

j) la vapeur précipitée dans le deuxième condenseur, éventuellement conjointement avec la décharge de fond de la colonne, est traitée par un passage supplémentaire ou est partiellement recyclée.

2. Procédé selon la revendication 1, caractérisé en ce qu'éventuellement on envoie 2 à 4 kg de vapeur d'eau surchauffée par 100 kg de glycérine brute par l'intermédiaire de l'évaporateur à film tombant dans la colonne.

3. Procédé selon les revendications 1 et 2, caractérisé en ce qu'on utilise des glycérines brutes contenant des sels aussi bien qu'exemptes de sels.

FIG. 1